# EUROPEAN PATENT SPECIFICATION

(11) **EP 1 987 840 B1**
(45) Date of publication and mention of the grant of the patent: **30.07.2014**
(21) Application number: 07008858.8
(22) Date of filing: 02.05.2007
(51) Int. Cl.: A61K 39/145, A61K 39/245, A61K 39/12

(54) **Prime-boost vaccine for the protection of equines against viral infection**
Prime-Boostimpfstoff zum Schutz von Pferden vor viraler Infektion
Vaccin de première immunisation pour la protection des chevaux contre les infections virales

(43) Date of publication of application: 05.11.2008
(73) Proprietor: Intervet International BV, 5831 AN Boxmeer (NL)
(72) Inventor: Van de Zande, Saskia, 5831 AN Boxmeer (NL)
(74) Representative: van Gent, Marieke

(56) References cited:
- EP-A- 1 779 866
- TOWNSEND H G G ET AL: "EFFICACY OF A COLD-ADAPTED, INTRANASAL, EQUINE INFLUENZA VACCINE: CHALLENGE TRIALS" EQUINE VETERINARY JOURNAL, R & W PUBLICATIONS, SUFFOLK, GB, vol. 33, no. 7, November 2001 (2001-11), pages 637-643, XP009063120 ISSN: 0425-1644
- CHAMBERS T M ET AL: "A NEW MODIFIED LIVE EQUINE INFLUENZA VIRUS VACCINE: PHENOTYPIC STABILITY, RESTRICTED SPREAD AND EFFICACY AGAINST HETEROLOGOUS VIRUS CHALLENGE" EQUINE VETERINARY JOURNAL, R & W PUBLICATIONS, SUFFOLK, GB, vol. 33, no. 7, November 2001 (2001-11), pages 630-636, XP009063106 ISSN: 0425-1644
- MINKE J M ET AL: "Equine viral vaccines: the past, present and future" VETERINARY RESEARCH, ELSEVIER, PARIS, NL, vol. 35, no. 4, July 2004 (2004-07), pages 425-443, XP002371664 ISSN: 0928-4249
- EUROPEAN MEDICINES AGENCY: "European Public Assessment Report, Equilis Prequenza Te (Intervet International B.V)" INTERNET CITATION, [Online] July 2005 (2005-07), XP002371662 Retrieved from the Internet: URL:http://www.emea.eu.int/vetdocs/PDFs/EP AR/equilisPrequenzaTe/20447005e n1.pdf> [retrieved on 2006] -& EUROPEAN MEDICINES AGENCY: "Equilis Prequenza Te" INTERNET CITATION, [Online] July 2005 (2005-07), XP002371663 Retrieved from the Internet: URL:http://www.emea.eu.in/vetdocs/vets/Epa r/equilisPrequenzaTe/equilisPre quenzaTeM.htm> [retrieved on 2006-03-09]
- SLATER J ET AL: "EQUINE IMMUNITY TO VIRUSES" VETERINARY CLINICS OF NORTH AMERICA. EQUINE PRACTICE, SAUNDERS, PHILADELPHIA, PA, US, vol. 16, no. 1, April 2000 (2000-04), pages 49-68, XP008006043 ISSN: 0749-0739
- BURKI F ET AL: "VIREMIA AND ABORTIONS ARE NOT PREVENTED BY TWO COMMERCIAL EQUINE HERPESVIRUS-1 VACCINES AFTER EXPERIMENTAL CHALLENGE OF HORSES", VETERINARY QUARTERLY, vol. 12, no. 2, 1990, pages 80-86, ISSN: 0165-2176
- BÜRKI F ET AL: "Attempts to immunoprotect adult horses, specifically pregnant mares, with commercial vaccines against clinical disease induced by equine herpesvirus-1.", ZENTRALBLATT FÜR VETERINÄRMEDIZIN. REIHE B. JOURNAL OF VETERINARY MEDICINE. SERIES B AUG 1991, vol. 38, no. 6, August 1991 (1991-08), pages 432-440, ISSN: 0514-7166
- HANNANT D ET AL: "Responses of ponies to equid herpesvirus-1 ISCOM vaccination and challenge with virus of the homologous strain", RESEARCH IN VETERINARY SCIENCE, vol. 54, no. 3, 1993, pages 299-305, XP009171474, ISSN: 0034-5288
- Jessett, Schrag and Mumford: "Protection provided by an attenuated EHV-1 vaccine against challenge with the virulent EHV-1 AB4 isolate" In: "Equine Infectious Diseases VIII", XP009171487, pages 414-415,

## Description

The present invention is concerned with vaccinating equines against viral infection. Equines are susceptible to a variety of infectious diseases. Due to the increasing international movement of horses the incidence of these diseases increases. Beside general sanitation, feeding, housing etc., vaccination of equines is therefore an important tool in preventing of animal suffering as well as the economic losses involved.

Infectious diseases for which horses can be vaccinated are, among others, equine influenza virus (EIV) and Equine herpes virus (EHV1 and EHV4) and Equine encephalitis (Western (WEE), eastern (EEE) and Venezuelan (VEE)) but also the West Nile virus, African horse Sickness, rabies, tetanus, Equine Infectious Anemia etc.

The vaccinations a given horse should receive depends on whether the horse is, for example, a domestic "backward" horse, where insect borne diseases, such as EEE, WEE and VEE, or WNV may be a problem, or a horse which travels a lot and may be exposed to may other horses, where contagious viruses such as EIV may be a problem. Most horses are vaccinated against tetanus, since tetanus causes against a deadly disease in horses. In case the horse is, for example, a broodmare, the risk of abortion, for example associated with EHV infection, should of course be minimized. Moreover, some diseases may only be a problem in a particular part of the world, such as the Potomac horse virus or rabies.

Vaccines may be based on either a modified live (attenuated), an inactivated pathogen or on a subunit vaccine based on one or more important immunogens derived from a pathogen. Vaccines exist for each pathogen separate, but also combination vaccines, for example for protection against EIV, EHV and tetanus, exist.

Equine herpes virus 1 (EHV-1) and equine herpes virus 4 (EHV-4) are an important cause of respiratory disease and abortion in horses world-wide. Especially when inactivated vaccines against EHV are used, the vaccine should contain a representative of both subtypes in order to provide adequate protection.

Such vaccines are commercially available, either alone, or in combination with, for example, influenza and tetanus, such as Equilis Resequin (Intervet International BV), which contains inactivated EHV-1 strain racH, inactivated EHV-4 strain 2252, and three inactivated EIV strains (representatives of the European and American type EIV).

A modified live vaccine for vaccination of equines against EHV infection, based on the attenuated EHV-1 strain racH, exist as well (Prevaccinol, Intervet International BV.

It has now been found that adequate protection against viral infection in equines can be achieved when vaccination with a live viral vaccine (prime) is followed by vaccination with an inactivated vaccine (boost) for the same virus, wherein the two shots are given no longer than 8 weeks apart.

The invention therefore provides a method for vaccination of equines against infection with equine herpes virus, wherein an animal is first vaccinated with a (prime) vaccine comprising an attenuated form of the pathogen, followed by a vaccination with a (boost) vaccine comprising the same pathogen in inactivated form, and wherein the boost vaccine is administered no longer than 8 weeks after the prime vaccine.

The invention further relates to the use of an inactivated equine viral pathogen, to prepare a boost vaccine for vaccinating equines that have been vaccinated with a priming vaccine, containing the same pathogen in an attenuated live form, no longer than 8 weeks prior to being vaccinated with the boost vaccine.

Preferably the two shots are given no longer than 6 weeks apart, for example, between 3-6 weeks apart, preferably between 4-6 weeks apart.

With "equine viral pathogen" is meant a virus that causes an infectious disease in equines. The method of the invention is especially useful for vaccination against viral infections such as infections with EHV-1.

The prime vaccine used in the present invention contains the viral pathogen in attenuated live form, meaning that the viral pathogen has been modified in such a way that ,it does not cause the disease, but still elicits an immune response in the vaccinated animal that attributes to protection against infection with the pathogen. In case of EIV the prime vaccine comprises an attenuated live equine influenza virus.

In case of a prime vaccine for EHV1 infection, the vaccine contains an attenuated EHV strain. An preferred example of an attenuated live EHV-1 strain that can be used in a prime vaccine as used with the method of the invention is the racH strain. This strain has served as the basis for modified live vaccines against EHV infection such as Prevaccinol (Intervet International BV). Such a vaccine can be used as the prime vaccine in the present invention. In a preferred embodiment of the invention the prime vaccine further contains an adjuvant.

The prime vaccine further contains the normal constituents of a modified live vaccine, such as a suitable pharmaceutical carrier which is usually a buffered diluent, optionally a preservative, etc., or any other suitable constituent known to the skilled person. The modified live vaccine may be administered via any suitable administration route.

The boost vaccine used with the invention contains the equine viral pathogen in an inactivated form.

A boost vaccine for use in a prime boost vaccination according to the invention, for the protection against EHV-1, may contain inactivated strains of both EHV-1 and EHV-4. Such inactivated vaccines against EHV are known in the art.

At least the boost vaccine used with the invention may contain at least two inactivated viral pathogens. For example, a combination vaccine may be used that contains both inactivated strains of EIV, as well as inactivated strains of EHV. Optionally the boost vaccine may contain further immunogens derived from other pathogens such as, for example, tetanus.
Inactivated combination vaccines against EIV/EHV & tetanus are known in the art (for example, Equilis Resequin and Equilis Resequin Plus (Intervet International BV).

The prime boost vaccination according to the invention can be applied to more than one pathogen at the same time.
For example, the prime vaccination for both pathogens may be given in the form of separate priming vaccinations, one for each pathogen, or in the form of a one-shot priming vaccination where attenuated live forms of different pathogens are combined in a combined live vaccine. For example, attenuated live strains of EIV and EHV may be combined in one and the same vaccine formulation, or may be give separately in two separate formulations, both used as priming vaccine in a method of the invention. The boost vaccination may then also be given in separate or combined form.
Either the boost or the prime vaccine may be combined in one and the same formulation with other immunogens such as, for example, purified tetanus toxoid, also when these other pathogens are not administered in a prime boost vaccine regimen according to the invention. Thus, for example, purified tetanus toxoid may be part of the boost vaccine. Thus, existing inactivated combination vaccines can be used as the boost vaccine in the present invention, when combined with a priming vaccination for one of the viral pathogens, which priming vaccination contains the pathogen in attenuated live form.

An inactivated boost vaccine as used in the invention may contain a suitable adjuvant. Suitable adjuvants are known in the art. For example, a suitable adjuvant may be based on Quillaja bark extracts or one or more saponin fractions thereof. Saponin fractions are produced from Quillaja bark extracts (Quil A) (Morein et al., Clin.Immunother., 3(6), 461-475,1995: "Immunostimulating Complexes, clinical potential in Vaccine Development"). Saponin fractions may be used as such, or in the form of a immunestimulatory complex such a s an ISCOM or ISCOM matrix, based on the saponins, a sterol and a lipid. Examples of suitable saponins fractions, and ISCOMs and matrices based thereon are given in Morein et al. (supra) and in WO96/11711. Useful fractions are for example "fraction An or "Fraction C" of Quil A or mixtures thereof. The "Equilis Prequenza" vaccine as developed by (Intervet International BV), is adjuvated with an Iscom matrix based adjuvant. Equilis Equenza (Intervet International BV) is adjuvated with Quil A.

Preferably both the prime(live) and boost vaccines are adjuvated with a saponins based adjuvant.

In a preferred vaccine combination according to the invention a vaccine containing live EHV strain racH, is used for the "prime" vaccination, in combination with a "boost" vaccine, said boost vaccine comprising inactivated EHV1/EHV4 virus, optionally combined with inactivated EIV strains (recommended EIV strains). Both the prime and the boost vaccine are advantageously adjuvated with an adjuvant based on Iscom matrices based on purified saponin fractions, such as "fraction An or "Fraction C" of Quil A or mixtures thereof, as mentioned above.

### EXAMPLES:

### Example 1: Different (EIV/1 EHV vaccination protocols against a challenge with EHV-1.

Twenty-five horses (with a low VN titre against EHV-1 and -4) were divided in 5 groups of 5 horses each.

Each horse was vaccinated IN or 1M according to Table 1. One dose 1 M (1 or 2ml) will be applied with a 2 ml syringe with green needle (0.8 x 40nm).

At t=O weeks horses in group A and 0 were vaccinated intranasally (IN) with attenuated live herpesvirus strain Rac H live vaccine (A and D). The horses in group C and 0 were vaccinated intramuscularly with Rac H inac vaccine containing a high antigenic mass, or with an inactivated EIV/EHV combination vaccine.

At T=4 weeks horses in groups C, 0 and E received a booster vaccination intramuscularly with an inactivated EIV/EHV combination vaccine (0 and E) or Rac H inac with high antigenic mass vaccine (C). Group F served as control group.

Body temperatures were measured and nasopharyngeal swabs were taken at day of vaccination. Blood samples for serology were taken at the time of vaccination, 7, 28 and 49 days after the first vaccination. Heparin blood samples were taken at each time of vaccination.

At 3 weeks after the second vaccination all horses were challenged intra nasally with EHV-1 strain M8. Starting 1 day before challenge, the horses will be observed daily for a period of 15 days for any clinical signs of herpesvirus. For the same period of time body temperatures will be measured and nasopharyngeal swabs will be taken daily for a period of 14 days after challenge. Blood samples for serology were taken at the time of challenge, and will be taken 7 and 14 days after challenge. Heparin blood samples were taken at time of challenge and will be every two days during 14 days.

### MATERIALS AND METHODS

### Vaccine formulations:

| | | |
|---|---|---|
| Formulation 1: | Live EHV vaccine (prevaccinol) | |
| Pharmaceutical form: | Liquid | |
| Composition: | Live EHV-1 (Rac H), 6.0log¹⁰TCID₅₀/ml vaccine | |
| | Adjuvant purified saponins 250 µg/ml vaccine | |
| | | |
| Formulation 2: | Rac H inac High AM | |
| Pharmaceutical form: | Liquid | |
| Composition: | EHV-1 (Rac H) 10000 AU/ml vaccine | |
| | Adjuvant: purified saponins 250 µg/ml vaccine | |
| | | |
| Formulation 3: | inac EIV/EHV combination vaccine | |
| Pharmaceutical form: | Liquid | |
| Composition: | South Africa/03 | 450 AU/ml vaccine |
| | Newmarket/02 | 450 AU/ml vaccine |
| | EHV-1 (Rac H) | 1000 AU/ml vaccine |
| | EHV-4 (2252) | 5000 AU/ml vaccine |
| | Tetanus toxoid | 40 LF/ml vaccine |
| | Adjuvant purified saponins | 250 µg /ml vaccine |
| | | |
| Challenge: | EHV-1 M8: | |
| Volume: | approximately 4 ml per animal | |
| Dosage: | 10^{7.5} EID₅₀ per animal | |
| Route: | 1 ml intranasal | |

**Table 1. Vaccination schedule**

| **Group (no. of horses)** | **1^{st} vaccination (t = 0) Vaccine volume/route** | **2^{nd} vaccination (t = 4 weeks Vaccine volume/route** | **Challenge (t = 7 weeks)** |
|---|---|---|---|
| A (5) | Rac H live 1ml IN | - | EHV-1 M8 |
| C (5) | Rac H Inac 1ml IM | Rac H Inac 1ml IM | EHV-1 M8 |
| D (5) | Rac H live 1ml IN | Inac EIV/EHV combi Resequin 2ml IM | EHV-1 M8 |
| E (5) | Inac EIV/EHV combi 2ml IM | Inac EIV/EHV combi 2ml IM | EHV-1 M8 |
| F (5) | - | - | EHV-1 M8 |

### Interpretation of results

Clinical observations, rectal temperatures and virus re-isolation data give an indication of the efficacy induced by the different EHV-1 vaccines against the EHV-1 challenge virus. Serology data, heparin blood samples and the nasal swabs taken at first and second vaccination and time of challenge confirm the negativity of the animals at the start of the challenge.

## Claims

1. Use of inactivated Equine Herpes virus 1 to prepare a boost vaccine for vaccinating Equines that have been vaccinated with a priming vaccine containing the same pathogen in an attenuated live form, no longer than 8 weeks prior to being vaccinated with the boost vaccine, and wherein the priming vaccine further contains an adjuvant and wherein the boost vaccine further contains an adjuvant.

2. Use according to claim 1 wherein the adjuvant is based on an ISCOM matrix.

## Patentansprüche

1. Verwendung von inaktiviertem equinem Herpesvirus 1 zur Herstellung eines Boost-Impfstoffs für die Impfung von Pferden, die nicht länger als 8 Wochen vor der Impfung mit dem Boost-Impfstoff mit einem den gleichen Krankheitserreger in abgeschwächter Lebendform enthaltenden Priming-Impfstoff geimpft worden sind, wobei der Priming-Impfstoff ferner ein Adjuvans enthält und wobei der Boost-Impfstoff ferner ein Adjuvans enthält.

2. Verwendung nach Anspruch 1, wobei das Adjuvans auf einer ISCOM-Matrix basiert.

## Revendications

1. Utilisation de virus de l'herpès équin 1 inactivé pour préparer un vaccin de rappel pour vacciner des équidés qui ont été vaccins avec une primovaccination contenant le même agent pathogène sous forme vivante atténuée, pas plus de 8 semaines avant d'être vaccinés avec le vaccin de rappel, et la primovaccination contenant en outre un adjuvant, et le vaccin de rappel contenant en outre un adjuvant.

2. Utilisation selon la revendication 1, l'adjuvant étant basé sur une matrice ISCOM.
